Europäisches Patentamt

European Patent Office

Office européen des brevets

(19)

(11) Publication number: **0 056 902**
**B1**

(12) **EUROPEAN PATENT SPECIFICATION**

(45) Date of publication of patent specification: **19.02.86**

(51) Int. Cl.⁴: **B 01 J 27/18,** B 01 J 23/22, C 07 C 51/25

(21) Application number: **81305884.9**

(22) Date of filing: **15.12.81**

(54) **Preparation of fluid bed-catalysts containing the mixed oxides of vanadium and phosphorus.**

(30) Priority: **31.12.80 US 221670**
**29.12.80 US 220624**

(43) Date of publication of application:
**04.08.82 Bulletin 82/31**

(45) Publication of the grant of the patent:
**19.02.86 Bulletin 86/08**

(84) Designated Contracting States:
**BE DE FR GB IT NL**

(56) References cited:
**US-A-4 016 105**
**US-A-4 092 269**
**US-A-4 132 670**
**US-A-4 179 404**

(73) Proprietor: **THE STANDARD OIL COMPANY**
**Midland Building**
**Cleveland, Ohio 44115 (US)**

(72) Inventor: **Milberger, Ernest Carl**
**34765 Sherwood Drive**
**Solon Ohio 44139 (US)**
Inventor: **Spitnale, Gregory Gene**
**3801 Willow Lake Drive**
**Kalamazoo Michigan 49008 (US)**
Inventor: **Lemanski, Michael Francis**
**812 London Road**
**Cleveland Ohio 44110 (US)**

(74) Representative: **Smith, Sydney et al**
**Elkington and Fife High Holborn House 52/54**
**High Holborn**
**London WC1V 6SH (GB)**

Courier Press, Leamington Spa, England.

**Description**

This invention relates to a method for preparing fluid bed catalysts useful in the production of dicarboxylic acid anhydrides by the oxidation of hydrocarbon. More particularly it is directed to the preparation of fluid bed catalysts suitable for producing maleic anhydride from 4-carbon atom hydrocarbons, such as n-butane, n-butenes, 1,3 butadiene or a mixture thereof.

The advantages of fluid bed hydrocarbon oxidation processes compared to fixed bed hydrocarbon oxidation processes are well known in the art, including the improvement of temperature control and heat transfer for oxidation reactions. Catalysts which are suitable for fixed bed processes are not necessarily suitable for fluid bed processes. Despite the incentive to utilize fluid bed technology in the production of maleic anhydride from 4-carbon atom hydrocarbons, there currently is no commercial fluid bed plant of this type in operation, all such commercial plants being fixed bed operations.

Catalysts containing vanadium and phosphorus oxides have been used in the oxidation of 4-carbon atom hydrocarbons, such as n-butane, n-butenes, 1,3 butadiene or mixtures thereof with molecular oxygen or oxygen-containing gas to produce maleic anhydride. Conventional methods of preparing these catalysts involve reducing a pentavalent vanadium compound, and combining the same with a phosphorus compound, and if desired, promoter element compounds under conditions which will provide vanadium in a valence state below +5 to form catalyst precursors capable of being converted to an oxide. The catalyst oxide precursor is then recovered and calcined, before or after the fixed bed catalyst particles are formed to provide active catalytic material.

U.S. Patents 3,888,886; 3,905,914; 3,931,046; 3,932,305 and 3,975,300 disclose the testing of promoted vanadium phosphorus oxide catalysts for maleic anhydride production from butane in one inch diameter fluid bed reactors. In most instances, the catalysts were prepared by forming the catalyst precursor in aqueous media (in 3,975,300 the precursor was formed in a paste of vanadium compound, a phosphorus compound and an organic reducing agent), drying and thereafter grinding and sieving the precursor to a powder of about 74 to 250 µm size. This manner of preparation, however, does not result in the uniform, microspheroidal catalyst particles preferred for successful fluid bed operation.

Commercial fluid bed catalysts are preferably microspheroidal particles within the range of 20 to 300 µm in average diameter, preferably having 80% of the particles within the range of 30 to 80 µm in diameter. Most preferably, 25 to 40% of the particles have an average diameter of less than 45 microns.

It is therefore an object of the invention to provide a process of preparing fluid bed oxidation catalysts containing a mixture of the oxides of vanadium and phosphorus.

The prepared fluid bed oxidation catalysts are useful in a process for producing maleic anhydride from 4-carbon atom hydrocarbons utilizing such catalysts.

We have found according to the invention that excellent uniform, microspheroidal vanadium phosphorus mixed oxide containing catalysts, useful in the production of maleic anhydride from 4-carbon atom hydrocarbons in fluid bed operations, can be obtained by spray drying an aqueous slurry of the catalyst precursor. Although this method of preparation may be utilized in forming fluid bed vanadium phosphorus mixed oxide catalysts prepared in aqueous media, it is unexpectedly efficacious when utilized in forming such catalysts in which the precursor was prepared in organic liquid solution or slurry.

It had previously been found that the preparation of vanadium phosphorus mixed oxide precursors in organic liquid media, solutions or slurries, particularly if maintained as essentially anhydrous, resulted in catalysts of high surface area and enhanced activity for the production of maleic anhydride from 4-carbon atom hydrocarbons. The presence of an excess of water in the final stages of preparation diminished both the surface area and activity of the catalysts. The formation of fluid bed catalysts by spray drying an organic liquid slurry of the catalyst precursor, because of flammability of the organic media, is not preferred.

We have found that once the vanadium-phosphorus mixed oxide precursor has been formed, it may be introduced into water to form an aqueous slurry which may then be spray dried to form microspheroidal fluid bed catalysts, without adversely affecting the surface area or activity of the catalyst.

According to the invention there is provided a process for the preparation of a fluid bed oxidation catalyst containing the mixed oxides of vanadium and phosphorus, comprising:

(a) preparing a catalyst precursor containing a vanadium phosphorus mixed oxide;

(b) comminuting the catalyst precursor;

(c) introducing the catalyst precursor into water prior or subsequent to comminution thereof to form an aqueous slurry; and

(d) spray drying said slurry to form microspheroidal catalyst particles.

Catalyst precursors of vanadium phosphorus mixed oxide catalysts for hydrocarbon oxidation may be prepared according to methods known in the art.

U.S. Patent No. 4,002,650 discloses the preparation of vanadium and phosphorus mixed oxide containing catalysts by reacting vanadium and phosphorus compounds in an aqueous solution, with HCl being utilized as a solvating and reducing agent for vanadium. Similar preparational techniques are described in EP—A—No. 3,431 in which the additional step of comminuting the vanadium-phosphorus precursor to a particle size of 500 to 700 µm (0.5 to 0.7 mm) is disclosed.

U.S. Patent No. 4,043,943 discloses the preparation of the catalyst precursor in a liquid organic medium, preferably anhydrous, wherein the vanadium compound is reduced and solvated by gaseous HCl followed by reaction with the phosphorus compound.

The preparation of oxidation catalysts containing the mixed oxides of vanadium and phosphorus is disclosed in EP—A—0031696 wherein a vanadium compound is at least partially solubilized in an organic liquid medium capable of reducing at least a portion of the vanadium to a +4 valence state, and unsolubilized vanadium having a particle size larger than about 0.1 mm diameter is removed from the medium before addition of a phosphorus-containing compound. The preparation of such catalysts is disclosed EP—A—39537 wherein partial reduction of a pentavalent vanadium compound is effected in the presence of a phosphorus compound in an organic liquid medium capable of reducing the vanadium.

The catalysts precursor may be recovered from the liquid reaction medium in which it was prepared (preferably an essentially anhydrous maintained organic liquid medium) by conventional methods, such as evaporation, filtration, centrifugation or decanting. The precursor is then preferably dried by heating. Alternatively, the recovered precursor, which is still partially wet with the organic liquid, may be treated with a low boiling solvent such as petroleum ether. In another embodiment, excess preparational reaction media may be substantially removed by vacuum filtration. In yet another embodiment, excess water can be introduced into the precursor containing organic liquid reaction medium, allowing an organic layer to separate from the aqueous layer.

After recovery, the catalyst precursor is comminuted before it is introduced into water to form an aqueous slurry. The catalyst precursor as produced generally has a particle size of greater than one micron average diameter before it is comminuted. It is preferred, however, that the catalyst precursor be reduced in particle size to less than one $\mu$m, and preferably less than one half $\mu$m average diameter. This step of comminuting may be accomplished before the precursor is recovered from its reaction media, or after recovery. Comminution after recovery can be effected either prior or subsequent to introduction into water. For example, dried catalyst precursor particles may be dry milled, such as by ball milling, or the catalyst precursor containing aqueous slurry may be ball milled.

The catalyst precursor preferably should be uncalcined when introduced into water. Substantial contacting of the calcined vanadium phosphorus mixed oxide catalyst with water (at less than 100°C) reduces the activity of the catalyst particularly if calcined in air.

The solids content of the catalyst precursor containing aqueous slurry should be adjusted to 25 to 60 weight percent. The catalyst precursor-containing aqueous slurry is then spray dried to form uniform, microspheroidal particles having a particle size range of between 20 to 300 $\mu$m generally between 20 to 240 $\mu$m. Spray drying may be accomplished by methods known in the art.

The catalyst precursor may contain promoter elements including but not limited to U, Co, Mo, Fe, Zn, Hf, Zr or mixtures thereof. These may be incorporated into the catalyst precursor in any of the methods known in the art, such as inclusion via the liquid reaction medium prior to or after reduction of the vanadium.

Inert diluents or supports may be added to the fluid bed catalyst, such as by addition of the diluent or support to the aqueous slurry prior to spray drying.

Catalysts suitable for the production of maleic anhydride from 4-carbon atom hydrocarbons generally have a phosphorus to vanadium ratio of 3:1 to 0.5:1 a P/V ratio of approximately 1.2:1 is preferred. These catalysts preferably exhibit an average valence for vanadium within the range of +3.5 to +4.6.

The catalyst may be calcined in air or an oxygen-containing gas at a temperature of 250°C. to 600°C. for a period of up to 5 hours or more. One method of calcination of the catalyst is accomplished by heating the catalyst in a mixture of steam and air or air alone over the catalyst at a temperature of 300°C. to 500°C. for a period of 1 to 5 hours. The catalyst may also be calcined in the presence of hydrocarbon, an inert gas, or both. The fluid bed catalyst prepared by the process of the present invention may be utilized in oxidation type fluid bed reactors known in the art.

The hydrocarbon reacted to form maleic anhydride may be n-butane, n-butenes, 1,3-butadiene, or a mixture thereof. It is preferred to use n-butane or a mixture of hydrocarbons that are produced in refinery streams. The molecular oxygen is most conveniently added as air, but synthetic streams containing molecular oxygen are also suitable. In addition to the hydrocarbon and molecular oxygen, other gases may be added to the reactant feed. For example, steam or nitrogen could be added to the reactants.

The ratio of the reactants may vary widely and is not critical. The ratio of molecular oxygen to the hydrocarbon may range from 3 to 30 moles of oxygen per mole of hydrocarbon. Preferred oxygen/hydrocarbon ratios are from 4 to 20 moles of oxygen per mole of hydrocarbon.

The reaction temperature may vary widely and is dependent upon the particular hydrocarbon and catalyst employed. Normally, temperatures of 250°C. to 600°C. are employed with temperatures of 325°C. to 500°C. being preferred. The contact time may be as low as a fraction of a second or as high as 50 seconds. The reaction may be conducted at atmospheric, superatmospheric or subatmospheric pressure. Operation at superatmospheric pressure is preferred, from greater than one atmosphere to about three atmospheres.

The following Examples illustrate the invention:—

The fluid bed catalyst described in Examples 1—8, below were used to produce maleic anhydride from n-butane in an 80 ml fluid bed reactor consisting of 35.5 cm length of stainless steel tubing having an outer diameter of 3.8 cm, having a stainless steel frit at the bottom of the tube to act as a gas (air) distributor and

**0 056 902**

an axial 1.3 ml outer diameter gas (hydrocarbon sparger/thermowell. The assembly contained an aluminium block pre-heater for the gases, and heating of the reactor unit was accomplished by placement in a temperature controlled molten salt bath.

The fluid bed catalyst described in Examples 9—47, below were used to produce maleic anhydride from n-butane in a 440 ml fluid bed reactor consisting of a 61 cm length of stainless steel tubing having an outer diameter of 3.8 cm having a stainless steel sparger at the bottom of the tube to act as a gas (air) distributor, with an axial 0.64 cm outer diameter thermowell and a separate hydrocarbon inlet at the bottom of the tube. The reactor was fitted with internal gas redistributing baffles. Gas preheating and reactor temperature control was accomplished by placement of the reactor unit in a thermostated fluidized sand bath.

Flasks for receiving the product maleic anhydride were air cooled, and tail gases were routed to a Carle Analytical Gas Chromatograph III for analysis. Reaction conditions and results of the tests run are described in Tables I through III. The results are stated in terms as follows:

$$\text{Single Pass Yield} = \frac{\text{Moles of Maleic Anhydride Formed}}{\text{Moles of Butane Fed}} \times 100$$

$$\text{Total Conversion} = \frac{\text{Moles of Butane Reacted}}{\text{Moles of Butane Fed}} \times 100$$

$$\text{Selectivity} = \frac{\text{Single Pass Yield}}{\text{Total Conversion}} \times 100$$

The throughput of hydrocarbon feed in the production of maleic anhydride, or the working rate imposed upon the catalyst is designated in the tables as WWH, or weight of feed/weight of catalyst/hour.

Examples 1—7

A fluid bed catalyst having the formula $V_{1.0}P_{1.2}Co_{0.2}O_x$, (where x=number of oxygens needed to satisfy the valence requirements of the other elements) was prepared by the following procedure. 1008 g vanadium pentoxide and 528.2 g cobaltous chloride dihydrate were added to 5.5 litres isobutanol with stirring to form a slurry. Anhydrous hydrogen chloride gas was bubbled through the liquid medium which was maintained by cooling at a temperature of about $20°\pm5°C$. After dissolution and reduction of the vanadium (about 4 hours, at which time the temperature of the now-homogenous red-brown solution began to drop) 1303 g 100% ortho-phosphoric acid in 2 litres isobutanol was added to the liquid medium. The liquid medium was then refluxed for 2 hours, acquiring a blue-green colour. The solution was dried by evaporation at a temperature of 150°C. The dried material was ground to yield a powdered catalyst precursor.

1500 g of the catalyst precursor powder was mixed with 3 litres water and the resulting slurry was milled for 12 hours. The comminuted catalyst precursor containing slurry was spray dried and calcined in air at 400°C for 16 hours. The resulting catalyst particles were dark green in colour and were microspheroidal in form. Results of the fluid bed (80 ml) production of maleic anhydride from n-butane using the catalyst of Examples 1—7 are listed in Table I.

Example 8

A fluid bed catalyst having the formula $V_{1.0}P_{1.2}Co_{0.2}O_x$ were prepared according to the procedure set forth in Examples 1—7 above, except that 935.5 g uranyl acetate dihydrate was substituted for the cobalt compound. Results of the fluid bed (80 ml) production of maleic anhydride from n-butane using the catalyst of Example 8 are listed in Table I.

Examples 9—20

A fluid bed catalyst having the formula $V_{1.0}P_{1.2}Co_{0.2}O_x$ was prepared according to the procedure set forth in Examples 1—7 above. Results of the fluid bed (440 ml) production of maleic anhydride from n-butane using the catalyst of Examples 9—20 are listed in Table II.

Examples 21—27

A fluid bed catalyst having the formula $V_{1.0}P_{1.2}O_x$ was prepared according to the following procedure. Catalyst precursor was prepared by introducing 7.276 kg $V_2O_5$, and 10.5 kg mixed phosphoric acid (including 1.2 kg $H_2O$) into 120 litres isobutanol with stirring, and refluxing the resulting slurry for 6 hours. The mixed phosphoric acid source contained 87% orthophosphoric acid, 11.5% pyrophosphoric acid and 1.5% triphosphoric acid based upon total weight of phosphoric acid. The slurry was cooled, the catalyst precursor recovered by filtration and dried for 3 hours at 150°C.

The dried catalyst precursor was ball milled for 5.5 hours, and 3000 g comminuted catalyst precursor

4

**0 056 902**

was thereafter introduced into 3667 g water with stirring. The resulting slurry was spray dried to yield uniform, microspheroidal catalyst particles. Results of the fluid bed (440 ml) production of maleic anhydride from n-butane using the catalyst of Examples 21—27 are listed in Table III.

Examples 28—37

A fluid bed catalyst having the formula 80 wt.% $V_{1.0}P_{1.2}O_x$ /20 wt.% $SiO_2$ was prepared according to the following procedure. 2.5 kg dry, comminuted catalyst precursor, prepared as in Examples 21—27 was introduced into 2.6 kg water, containing 1.8 kg Nalco 1034A silica sol (trade designation of Nalco Chemical Co.), with stirring. The resulting slurry was spray dried to yield uniform, microspheroidal catalyst particles. Results of the fluid bed (440 ml) production of maleic anhydride from n-butane using the catalyst of Examples 28—37 are listed in Table III.

Examples 38—47

A fluid bed catalyst having the formula 70 wt.% $V_{1.0}P_{1.2}O_x$ /30 wt.% $SiO_2$ was prepared according to the following procedure. Catalyst precursor was prepared by introducing 7.276 kg $V_2O_5$ and 9.39 kg orthophosphoric acid (100%) in 120 litres isobutanol with stirring, and refluxing the resulting slurry for 16 hours. The slurry was cooled, the catalyst precursor recovered by filtration and dried for 2 hours at 150°C.

The dried catalyst precursor was ball milled for 24 hours, and 884 g comminuted catalyst precursor was introduced into 2000 g water containing 1063 g Nalco 1034A silica sol, with stirring. The resulting slurry was spray dried to yield uniform, microspheroidal catalyst particles. Results of the fluid bed (440 ml) production of maleic anhydride from n-butane using the catalyst of Examples 38—47 are listed in Table III.

As can be seen from the results listed in Table I through III, fluid bed catalysts containing the mixed oxides of vanadium and phosphorus may be prepared according to the present invention, such catalysts being useful in the production of maleic anhydride from 4-carbon atom hydrocarbons. The fluid bed catalysts thus prepared are uniform, microspheroidal, and are suitable for use as commercial fluid bed catalysts, a substantial portion of the microspheroidal particles of such catalysts having particle sizes within the range of 20 μm to 100 μm. The particle size requirement for fluid bed catalysts, set forth above, are met by these catalysts. Catalyst precursors which have been prepared in organic media, unexpectedly retain their high activity for the production of maleic anhydride, when further treated according to the process of the present invention.

TABLE I

Fluid bed (80 ml) production of maleic anhydride from n-butane

| Example No. | Temperature °C Bath | Bed | Ratio air/ hydrocarbon | Contact time (sec) | WWH | % Conversion | % Yield | Maleic anhydride % Selectivity | Time on stream (hrs.) |
|---|---|---|---|---|---|---|---|---|---|
| $V_{1.0}P_{1.2}Co_{0.2}O_x$ Catalyst | | | | | | | | | |
| 1 | 389 | 390 | 66.7 | 6.6 | 0.015 | 92.0 | 58.6 | 63.7 | 66 |
| 2 | 389 | 390 | 64.0 | 6.6 | 0.016 | 76.6 | 48.8 | 63.7 | 165 |
| 3 | 375 | 373 | 72.3 | 6.2 | 0.011 | 93.9 | 64.1 | 68.3 | 72 |
| 4 | 375 | 373 | 61.8 | 6.0 | 0.014 | 86.1 | 58.5 | 68.0 | 139 |
| 5 | 376 | 375 | 65.7 | 6.0 | 0.013 | 89.0 | 58.8 | 66.5 | 166 |
| 6 | 377 | 375 | 67.0 | 6.1 | 0.012 | 79.6 | 50.5 | 63.6 | 265 |
| 7 | 375 | 375 | 65.8 | 6.1 | 0.013 | 81.7 | 52.6 | 64.7 | 310 |
| $V_{1.0}P_{1.2}U_{0.2}O_x$ Catalyst | | | | | | | | | |
| 8 | 397 | | 78.4 | 6.1 | | 95 | 52.8 | 55.7 | |

TABLE II

Fluid bed (440 ml) production of maleic anhydride from n-butane using $V_{1.0}P_{1.2}Co_{0.2}O_x$ catalyst

| Example No. | Temperature °C Bath | Bed | Ratio air hydrocarbon | Contact time (sec.) | WWH | % Conversion | Maleic anhydride % Yield | % Selectivity | Time on stream (hrs.) |
|---|---|---|---|---|---|---|---|---|---|
| 9 | 371 | 380 | 61.9 | 5.84 | 0.015 | 91.6 | 59.5 | 65.0 | 24 |
| 10 | 377 | 385 | 64.6 | 5.20 | 0.016 | 81.3 | 56.5 | 69.5 | 134 |
| 11 | 376 | 383 | 64.6 | 5.20 | 0.016 | 74.8 | 50.6 | 67.6 | 240 |
| 12 | 378 | 386 | 59.9 | 6.08 | 0.014 | 75.8 | 52.8 | 69.6 | 328 |
| 13 | 394 | 398 | 59.8 | 5.86 | 0.015 | 80.6 | 55.8 | 69.3 | 435 |
| 14 | 411 | 422 | 40.4 | 6.15 | 0.019 | 92.4 | 59.2 | 64.1 | 647 |
| 15 | 409 | 420 | 39.3 | 7.04 | 0.017 | 87.0 | 57.0 | 65.5 | 834 |
| 16 | 410 | 422 | 39.6 | 6.95 | 0.018 | 90.6 | 56.4 | 62.3 | 1,026 |
| 17 | 416 | 428 | 26.6 | 8.53 | 0.021 | 76.3 | 49.0 | 68.8 | 1,290 |
| 18 | 400 | 409 | 61.5 | 5.31 | 0.016 | 85.2 | 54.6 | 64.1 | 1,481 |
| 19 | 409 | 422 | 35.5 | 5.19 | 0.027 | 74.9 | 70.4 | 52.8 | 1,630 |
| 20 | 431 | 443 | 39.0 | 6.79 | 0.018 | 93.2 | 57.0 | 61.2 | 1,847 |

## TABLE III
### Fluid bed (440 ml) production of maleic anhydride from n-butane using $V_{1.0}P_{1.2}O_x$ catalysts

| Example No. | Temperature °C Bath | Bed | Ratio air/ hydrocarbon | Contact time (sec.) | WWH | % Conversion | Maleic anhydride % Yield | % Selectivity | Time on stream (hrs.) |
|---|---|---|---|---|---|---|---|---|---|
| $V_{1.0}P_{1.2}O_x$ Catalyst | | | | | | | | | |
| 21 | 386 | 394 | 29.0 | 6.7 | 0.020 | 97.9 | 60.0 | 61.2 | 78 |
| 22 | 394 | 405 | 29.5 | 6.8 | 0.020 | 83.3 | 55.8 | 67.0 | 145 |
| 23 | 403 | 411 | 36.4 | 9.1 | 0.020 | 82.2 | 57.6 | 70.0 | 196 |
| 24 | 426 | 440 | 26.4 | 8.6 | 0.028 | 89.8 | 56.4 | 62.8 | 246 |
| 25 | 425 | 439 | 29.8 | 8.8 | 0.024 | 91.0 | 55.9 | 61.4 | 334 |
| 26 | 425 | 440 | 28.9 | 8.9 | 0.024 | 93.3 | 57.1 | 61.2 | 429 |
| 27 | 425 | 442 | 22.4 | 10.8 | 0.026 | 92.4 | 53.6 | 58.0 | 479 |
| 80% $V_{1.0}P_{1.2}O_x$ /20% $SiO_2$ | | | | | | | | | |
| 28 | 376 | 380 | 61.7 | 6.0 | 0.014 | 88.0 | 41.5 | 47.1 | 43 |
| 29 | 405 | 414 | 27.6 | 5.7 | 0.031 | 78.3 | 46.8 | 59.8 | 257 |
| 30 | 431 | 439 | 27.4 | 5.6 | 0.030 | 76.2 | 46.8 | 61.4 | 527 |
| 31 | 438 | 446 | 28.4 | 8.5 | 0.019 | 94.4 | 50.7 | 53.7 | 720 |
| 32 | 438 | 446 | 28.8 | 8.2 | 0.019 | 93.1 | 51.0 | 54.7 | 882 |
| 33 | 438 | 446 | 28.1 | 8.5 | 0.019 | 91.5 | 51.9 | 56.7 | 957 |
| 34* | 438 | 449 | 28.5 | 10.9 | 0.024 | 94.6 | 50.9 | 53.8 | 1,074 |
| 35* | 438 | 449 | 31.2 | 11.1 | 0.023 | 95.6 | 46.0 | 48.2 | 1,285 |
| 36* | 438 | 451 | 27.0 | 8.9 | 0.031 | 86.4 | 48.1 | 55.7 | 1,375 |
| 37* | 439 | 451 | 24.0 | 10.0 | 0.031 | 86.3 | 46.2 | 53.6 | 1,400 |

*Reactor outlet pressure 10 PSIG (1,68 bars)

TABLE III (continued)

| Example No. | Temperature °C Bath | Bed | Ratio air/ hydrocarbon | Contact time (sec.) | WWH | % Conversion | Maleic anhydride % Yield | % Selectivity | Time on stream (hrs.) |
|---|---|---|---|---|---|---|---|---|---|
| 70% $V_{1.0}P_{1.2}O_x$ /30% $SiO_2$ | | | | | | | | | |
| 38 | 358 | 367 | 58 | 7.0 | 0.014 | 90.5 | 51.9 | 57.4 | 114 |
| 39 | 359 | 369 | 58 | 7.0 | 0.014 | 91.4 | 52.3 | 57.2 | 138 |
| 40 | 360 | 368 | 58 | 7.0 | 0.014 | 90.9 | 53.7 | 59.1 | 210 |
| 41 | 360 | 375 | 29.7 | 9.1 | 0.021 | 78.6 | 46.3 | 58.9 | 329 |
| 42 | 371 | 384 | 28.8 | 9.8 | 0.019 | 78.4 | 45.5 | 58.1 | 659 |
| 43 | 370 | 382 | 26.8 | 9.8 | 0.020 | 76.2 | 48.9 | 64.2 | 775 |
| 44 | 382 | 394 | 27.8 | 9.6 | 0.020 | 77.2 | 45.0 | 58.3 | 965 |
| 45 | 380 | 393 | 28.7 | 9.8 | 0.019 | 76.8 | 44.4 | 57.8 | 1,085 |
| 46 | 380 | 392 | 27.3 | 8.7 | 0.021 | 72.6 | 44.0 | 60.6 | 1,443 |
| 47 | 380 | 388 | 37.6 | 9.4 | 0.014 | 77.6 | 46.7 | 60.1 | 1,826 |

**Claims**

1. A process for the preparation of a fluid bed oxidation catalyst containing the mixed oxides of vanadium and phosphorus, comprising:

(a) preparing a catalyst precursor containing a vanadium phosphorus mixed oxide;

(b) comminuting the catalyst precursor;

(c) introducing the catalyst precursor into water prior or subsequent to comminution thereof to form an aqueous slurry; and

(d) spray drying said slurry to form microspheroidal catalyst particles.

2. A process as claimed in claim 1 characterised in that it includes an additional step of calcining the microspheroidal catalyst particles.

3. A process as claimed in claim 1 or claim 2 characterised in that the precursor is prepared in an organic liquid.

4. A process as claimed in claim 1 or claim 2 characterised in that the catalyst precursor is prepared in an organic liquid slurry.

5. A process as claimed in any of claims 1 to 4 characterised in that the catalyst precursor is substantially dried prior to introducing it into water.

6. A process as claimed in any of claims 1 to 5 characterised in that said catalyst precursor is comminuted to a particle size of less than one µm average diameter.

7. A process as claimed in any of claims 1 to 6 characterised in that the slurry has a solids content of 25 to 60 weight percent.

8. A process as claimed in any of claims 1 to 7 characterised in that said micropheroidal particles has an average particle size of less than 300 µm, preferably from 20 to 240 µm.

9. A process as claimed in any of claims 1 to 8 in which the precursor additionally comprises promoter elements selected from the group consisting of U, Co, Mo, Fe, Zn, Hf, Zr and mixtures thereof.

**Patentansprüche**

1. Verfahren zum Herstellen eines Fließbettkatalysators, der die gemischten Oxide von Vanadium und Phosphor enthält, dadurch gekennzeichnet, daß

a) ein Katalysatorvorläufer hergestellt wird, der ein gemischtes Vanadium-Phosphor-Oxid enthält;

b) der Katalysatorvorläufer pulverisiert wird;

c) der Katalysatorvorläufer vor oder nach dem Pulverisieren desselben in Wasser eingegeben wird, um einen wässerigen Brei zu bilden; und

d) der Brei durch Zerstäuben getrocknet wird, um mikrokugelige Katalysatorpartikel zu bilden.

2. Verfahren nach Anspruch 1, dadurch gekennzeichnet, daß die mikrokugeligen Katalysatorpartikel zusätzlich kalziniert werden.

3. Verfahren nach Anspruch 1 oder 2, dadurch gekennzeichnet, daß der Vorläufer in einer organischen Flüssigkeit erzeugt wird.

4. Verfahren nach Anspruch 1 oder 2, dadurch gekennzeichnet, daß der Katalysatorvorläufer in einem organischen flüssigen Brei erzeugt wird.

5. Verfahren nach einem der Ansprüche 1 bis 4, dadurch gekennzeichnet, daß der Katalysatorvorläufer vor dem Eingeben in Wasser im wesentlichen getrocknet wird.

6. Verfahren nach einem der Ansprüche 1 bis 5, dadurch gekennzeichnet, daß der Katalysatorvorläufer auf eine Partikelgröße mit einem durchschnittlichen Durchmesser von weniger als 1 µm zerkleinert wird.

7. Verfahren nach einem der Ansprüche 1 bis 6, dadurch gekennzeichnet, daß der Brei einen Feststoffgehalt von 25 bis 60 Gew.% hat.

8. Verfahren nach einem der Ansprüche 1 bis 7, dadurch gekennzeichnet, daß die mikrokugeligen Partikel eine durchschnittliche Partikelgröße von weniger als 300 µm, vorzugsweise von 20 bis 240 µm, aufweisen.

9. Verfahren nach einem der Ansprüche 1 bis 8, dadurch gekennzeichnet, daß der Vorläufer zusätzlich Beschleunigerelemente aus der Gruppe, die aus U, Co, Mo, Fe, Zn, Hf, Zr und Gemischen derselben besteht, enthält.

**Revendications**

1. Procédé de préparation d'un catalyseur d'oxydation pour lit fluidisé contenant les oxydes mixtes de vanadium et de phosphore, consistant à:

a) préparer un précurseur de catalyseur contenant un oxyde mixte de vanadium et de phosphore;

b) broyer finement le précurseur de catalyseur;

c) introduire le précurseur du catalyseur dans de l'eau avant ou après le broyage fin de celui-ci, pour former une bouillie aqueuse;

d) sécher par pulvérisation ladite bouillie pour former des particules de catalyseur microsphéroïdales.

2. Procédé selon la revendication 1, caractérisé par le fait qu'il comprend une étape supplémentaire de calcination des particules de catalyseur microsphéroïdales.

3. Procédé selon la revendication 1 ou la revendication 2, caractérisé par le fait que le précurseur est préparé dans un liquide organique.

4. Procédé selon la revendication 1 ou la revendication 2, caractérisé par le fait que le précurseur du catalyseur est préparé dans une bouillie de liquide organique.

5. Procédé selon l'une des revendications 1 à 4, caractérisé par le fait que le précurseur de catalyseur est sensiblement séché avant son introduction dans l'eau.

6. Procédé selon l'une des revendications 1 à 5, caractérisé par le fait que le précurseur de catalyseur est broyé finement à une taille de particules inférieure à 1 µm de diamètre moyen.

7. Procédé selon l'une des revendication 1 à 6, caractérisé par le fait que la bouillie a une teneur en matières solides de 25 à 60% en poids.

8. Procédé selon l'une des revendications 1 à 7, caractérisé par le fait que les particules microsphéroïdales ont une taille moyenne de particules inférieure à 300 µm de préférence de 20 à 240 µm.

9. Procédé selon l'une des revendications 1 à 8, dans lequel le précurseur comprend en outre des éléments promoteurs choisis dans le groupe formé par U, Co, Mo, Fe, Zn, Hf, Zr et leurs mélanges.